Europäisches Patentamt

**European Patent Office**

**Office européen des brevets**

(11) Publication number: **0 030 087**

**A1**

## EUROPEAN PATENT APPLICATION

(21) Application number: **80303993.2**

(22) Date of filing: **07.11.80**

(51) Int. Cl.³: **G 01 N 33/54**

(30) Priority: **13.11.79 GB 7939213**

(43) Date of publication of application:
**10.06.81 Bulletin 81/23**

(84) Designated Contracting States:
**DE FR GB IT**

(71) Applicant: **TECHNICON INSTRUMENTS COMPANY LIMITED**
**Evans House Hamilton Close**
**Basingstoke Hants(GB)**

(72) Inventor: **Smith, David Stuart**
**263-269 City Road**
**London EC1V 1JX(GB)**

(74) Representative: **Wain, Christopher Paul et al,**
**A.A. THORNTON & CO. Northumberland House 303-306**
**High Holborn**
**LONDON WC1V 7LE(GB)**

(54) Immunoassay method and apparatus and kit for carrying out the method.

(57) The quantitative immunoassay of an analyte (e.g. antigen or antibody) in a fluid is effected using an excess of magnetically attractable particles bearing a receptor which selectively binds to the analyte. After separation of the analyte-carrying particles from the reaction mixture, the excess receptor or the amount of bound analyte is assayed, preferably using a colourimetric or fluorescent label. On a manual basis, the whole assay may be conducted in a single test-tube. Alternatively, automated continuous flow techniques may be used, for which purpose a continuous flow analyser is described. Among the advantages of the method of the invention are the avoidance or reduction of serum interferences and the provision of a simple manual assay technique.

0030087

"IMMUNOASSAY METHOD AND APPARATUS"

This invention relates to a method of assaying a liquid for a constituent of interest (analyte) therein, and is particularly (but not exclusively) concerned with a method of immunoassay and apparatus useful therefor.

It is well known in the art of immunoassays that a liquid sample to be analysed for an analyte therein will often contain one or more other substances capable of interfering in the assay procedure and thus causing error. Various ways are known for avoiding or reducing this problem. For example, substances can be added to the sample to neutralise or destroy the interfering species, but this is not always a very efficient or convenient way of dealing with the problem.

We have now devised a method of immunoassay, and apparatus useful therefor, by which this problem can be overcome or at least substantially reduced.

In one aspect, the invention provides a method of quantitatively assaying a liquid for an analyte therein which comprises the steps of (a) mixing a sample of the liquid with finely divided magnetically attractable particles comprising a reagent, and then (b) separating the particles from the mixture,

characterised in that:

i)      the said reagent is a receptor which selectively binds with the analyte in the mixture, the amount of receptor used in step (a) being in excess of that required to bind in excess of that required to bind with the analyte in the mixture;

ii)     the particles separated in step (b) have analyte bound thereto;   and

iii)    the method includes the further step

(c)    assaying the receptor excess or the amount of analyte bound to the particles and therefrom determining the amount of analyte in the liquid sample.

In step (a) of the method, the liquid sample is mixed with magnetically attractable particles. The particles comprise  a receptor which will selectively bind with the analyte under assay. Thus, the analyte becomes bound to the particles, but any interfering species present does not, or does not to any significant extent, become bound to the particles. The quantity of particles added is such as to provide an excess of receptor so that the maximum amount of analyte is extracted from the liquid sample. The excess receptor may, according to one feature of the invention, be "back titrated" in step (c) to determine the amount of analyte which has become bound in step (a).

We believe that, in most circumstances, all or substantially all of the analyte in the sample will become bound to the particles in step (a). It is possible, however, that sometimes an equlibrium set up between bound analyte and free analyte will result in not quite all of the analyte becoming bound to the particles. This does not invalidate the assay, however, since the results obtained in step (c) may be compared with standard results of assays made on liquid samples containing known concentrations of analyte. In this way, accurate determinations of unknown analyte concentrations can be made.

The nature of the magnetic particles themselves is not critical. Magnetic particles for use in immunoassays are known and are commercially available. They normally comprise a polymeric matrix in which is embedded magnetically attractable material (such as $Fe_3O_4$). The nature of the receptor will depend upon the analyte which is to be assayed. Where, for example, the analyte is an antigen or hapten, the receptor will normally be the corresponding immunospecific antibody or a similar binding protein. Conversely, where the analyte is an antibody or other binding protein, the receptor can be a corresponding antigen or hapten.

The receptor is immobilised on or in the magnetic particles in such manner as to be accessible to the analyte. It may, for example, be covalently bound to the particles, or bound thereto by physico-chemical bonds, or merely embedded therein. It is essential, however, that the receptor should not break free of the particles during the assay.

Preferably, the magnetically attractable particles (with the receptor immobilised thereon) will have a specific gravity close to that of the liquids in which they are suspended during the assay. This reduces the amount of sinking or flotation and renders the suspensions more stable, so improving the contact between the particles and the suspending liquids. Normally, specific gravities in the range 1.4 to 3.2 are preferred.

The liquid sample under assay can be a biological fluid, such as blood serum or urine, or a liquid of non-biological origin. Additionally, it may be a liquid formed after treatment of blood serum or urine (for example) with other reagents. Thus, for example, an antigen or antibody in blood serum could first be reacted to form a complex, and then the method of the invention applied to assay the complex using, for example, RF or C1q as receptor.

Step (b) of the method of the invention involves separation of the analyte-bearing particles from the mixture formed in step (a). This is most conveniently effected by applying a magnetic field to hold the particles localised whilst the liquid is removed. In manual assays, the particles can for example be sedimented using a magnet, and the supernatant liquid aspirated or decanted. In automated continuous flow assays, the particles can be held by a magnetic field in a conduit whilst the liquid continues to flow on, leaving the particles behind. If desired, the particles can be washed one or more times before step (c). It will be seen that, by separating the particles from the liquid, the analyte under assay is separated from any interfering species present in the liquid. Moreover, this separation can be effected very efficiently and simply, without the need for the addition of any reagents to destroy or inactivate the interfering species. The use of magnetic particles in this way also has the advantage of concentrating the analyte under assay which, in turn, enhances the sensitivity of the assay. Furthermore, according to a preferred feature of the invention (to be described hereinafter), the particles themselves can be simply tested to determine the amount of analyte present.

In step (c) of the method of the invention, either the receptor excess (i.e. the binding capacity of the receptor not utilised in binding analyte in step (a)) is assayed, or the amount of analyte on the particles is assayed, to provide a measure of the amount of analyte present in the original sample. There are various ways in which these assays can be performed. We prefer, however, to make use of a labelled substance. For example, to measure the receptor excess, the particles may be suspended in a liquid containing a labelled substance which will bind with the receptor (but not with the bound analyte). The amount of labelled substance which becomes bound to the particles, or which remains unbound in the liquid, is then measured and the amount of analyte thus determined. It is preferred to measure the free labelled substance rather than the bound labelled substance.

A similar procedure can be used to measure the bound analyte, except that in this case the labelled substance is one which will bind with the analyte but not with the receptor. Again, we prefer to measure the amount of label remaining unbound in the liquid although it is possible to measure the amount which has become bound to the analyte on the particles.

The nature of the labelled substance is critical only in relation to its binding properties. Where it has to bind with an antigen or hapten (as receptor or analyte), a labelled immunospecific antibody or closely similar binding protein will generally be used. Where the receptor or hapten is an antibody, the corresponding labelled antigen or hapten (or closely similar compound) will generally be used.

The label may be any readily identifiable group such as a radioactive, fluorimetric, chemiluminescent or enzymic group. For assays conducted on a discrete manual basis, we prefer to use fluorimetric labels.

In the two step (c) assay procedures described above, the analyte substantially remains bound to the particles throughout. This is a preferred feature of the invention, but it is alternatively possible to elute the analyte from the particles and assay it directly in solution by conventional techniques, e.g. by a competitive binding assay. This procedure is not generally preferred because it is normally more time-consuming and, because extra steps may be involved, can be less accurate.

It is possible, in certain circumstances, to assay either the receptor excess or the bound analyte by mixing the particles with one or more substances with which the receptor excess or analyte will react (without becoming bound). They can then be assayed by the extent of any such reaction which may, for example, by an enzymic reaction in which the receptor excess or analyte (but not both) is a reactant.

In step (c) of the method of the invention, it will usually be necessary to separate the particles from the liquid reaction mixture, e.g. to assay the label or to measure the extent of a reaction. Such a separation step can be effected in a similar manner to that in step (b) of the method. Where, however, the method of the invention is effected on a discrete manual basis, using fluorimetric or chemiluminescent labels for example, this complete separation step can be avoided. In this case, the magnetic particles may be drawn out of suspension (e.g. sedimented) and held localised within the reaction vessel by a magnetic field, and the clear liquid remaining can be directly assayed (through light-transmitting walls of the vessel) for its fluorimetric or chemiluminescent label content. This is a highly advantageous preferred feature of the invention since it greatly facilitates manual assay. There is also the further advantage that the whole assay can be conducted in a single vessel.

The method of the invention can be carried out on an automated continuous flow basis. Thus, the mixture formed in step (a) is flowed along a conduit and the separation in step (b) is effected using one or more magnetic traps disposed in the conduit. The trapped particles may if desired be washed and then released for further travel in the conduit, during which the assay for receptor excess or bound analyte (with or without an elution step) is effected. Where this assay is effected using a labelled substance, the particles may be separated from the reaction liquid using one or more magnetic traps, and the liquid or the particles assayed for label in conventional manner.

The invention thus includes a method of analysing a plurality of fluid samples for analytes therein, which comprises:

i)     flowing a series of reaction mixtures in turn and on a discrete basis sequentially along a conduit, each reaction mixture comprising a sample of the liquid to be analysed for analyte and magnetically attractable particles comprising a receptor, the receptor being capable of selectively binding with the analyte and being present in an amount in excess of that required to bind with the analyte in each mixture;

ii)    magnetically trapping the particles in each reaction mixture to hold the particles against flow and thereby to separate them from the mixture; and

iii)   determining the receptor excess or the analyte bound to the particles ( preferably without eluting the analyte from the particles) and thereby determining the analyte in each sample.

Preferably, step (iii) comprises forming a mixture of the particles with a substance capable of reacting selectively either with the receptor excess or with the bound analyte and then assaying the extent of reaction. Most preferably, a labelled substance is used which selectively binds either with the receptor excess or with the analyte on the particles, and the particles or liquid are then assayed for label.

The invention also includes apparatus for carrying out the method on a continuous flow basis, which apparatus comprises means for flowing a plurality of reaction mixtures in turn along a conduit on a discrete basis (each reaction mixture comprising the particles and the liquid sample as described above); first trap means for magnetically trapping the particles in the conduit in each reaction mixture to hold them against the flow and so separate them from their respective mixture; means for mixing the separated particles with a liquid reagent; second trap means for separating the particles from said liquid reagent; and means for assaying the separated particles or the liquid to thereby enable determination of the amount of analyte in each sample. Preferably, the liquid reagent comprises a labelled substance which binds either to the receptor excess, or to the analyte, on the particles. Downstream of the second trap, either the particles or the liquid are then assayed for label.

In carrying out the method of the invention, it is usual to obtain an absolute measure of the amount of analyte by comparing the results obtained with standard results or standard curves. The standard results or curves are obtained by carrying out the method using solutions of known concentrations of the analyte in question, and thus constructing standard curves relating the concentration of analyte to the final reading obtained in the assay. The final reading in the assay will of course depend on the precise method used, i.e. it may be a fluorimetric or radioactivity reading made on the liquid or the particles, or it may be some other parameter. The use of standard results to give absolute values in this way is well known and understood in the art.

The method of the invention is useful for the assay of a wide variety of analytes. Of principal interest, however, are the thyroid hormones ($T_4$ and $T_3$), drugs such as digoxin and phenytoin, steroids such as oestriol, and pregnancy associated proteins such as human placental lactogen.

In order that the invention may be more fully understood, reference is made to the accompanying drawings, in which:

FIGURE 1 illustrates schematically one embodiment of manual assay according to the invention;

FIGURE 2 illustrates schematically one form of continuous flow apparatus for carrying out an embodiment of the method on an automated basis; and

FIGURE 3 is a standard curve referred to hereinafter in the Example.

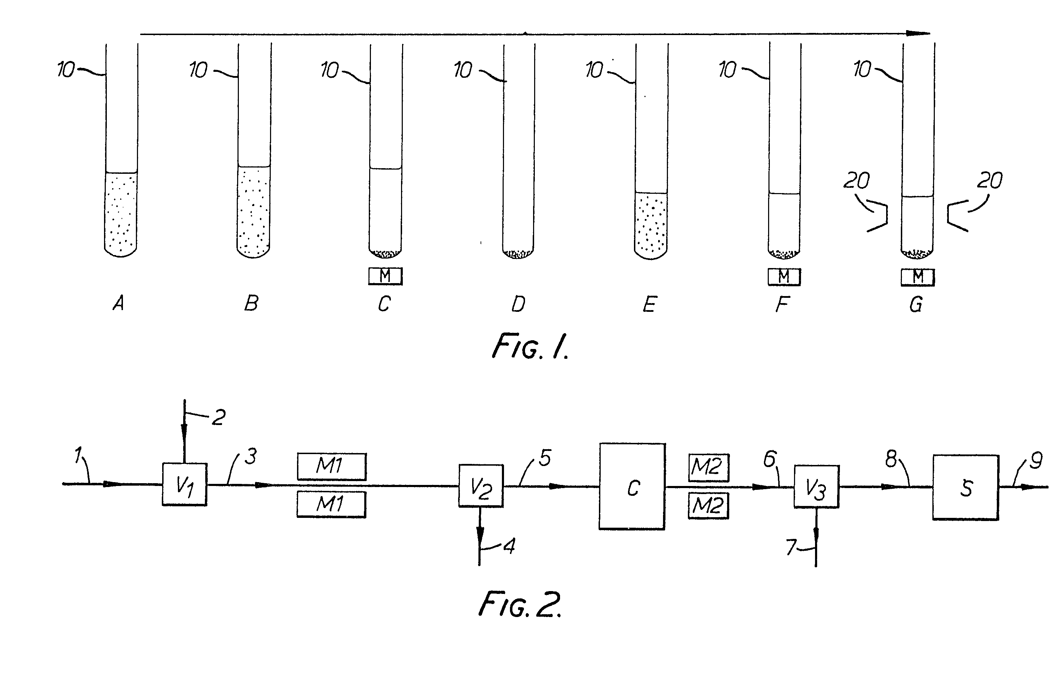

Referring to Figure 1, the assay is shown as comprising seven stages A to G, each conducted in the same test tube 10. In stage A, a suspension of finely divided magnetic particles, in measured amount, is placed in tube 10. The particles comprise receptor. In step B, the liquid sample under assay containing analyte is added. The mixture equilibrates, and then a magnet M is placed under the tube (stage C) and the particles are caused to sediment to the bottom of the tube. The supernatant liquid is then removed leaving the particles in the tube (stage D). A liquid is now added to the tube, which liquid contains a labelled substance which will bind with either the analyte or the receptor (but not both) and the magnetic particles are suspended in the liquid (step E). The labelled substance is (for example) fluorimetrically labelled. After equilibration, the magnet M is again placed beneath the tube to sediment the particles leaving a clear supernatant (step F). Finally, the supernatant is assayed (using device 20) through the test-tube wall whilst the magnetic particles are held clear of the viewing area.

Figure 2 illustrates schematically one arrangement for effecting the method of the invention on a continuous flow basis. A mixture of the magnetic particles and liquid sample under assay is flowed (in the arrow direction) along conduit 1 and through valve

$V_1$ into conduit 3. The mixture then passed between electromagnets $M_1$ (constituting a first magnetic trap) and the particles are retained whilst the liquid flows on to valve $V_2$, where it is diverted to waste down conduit 4. The valve $V_1$ is now altered to close conduit 1 and allow liquid containing a labelled substance to flow from conduit 2 into conduit 3. As this liquid flows through the magnetic trap, the magnetic field is removed and the particles are carried out of the trap by the liquid. Valve $V_2$ is changed to close conduit 4, and the liquid/particle suspension flows through mixing coil C where equilibration occurs. From coil C, the mixture passes through a second magnetic trap $M_2$, in which the particles are retained whilst the liquid flows on in conduit 6. If a label measurement is to be made on the liquid, valve $V_3$ closes conduit 7 and the liquid flows directly along conduit 8 to the counter S from whence it exits to waste through conduit 9. If, however, the label assay is to be made on the particles, the liquid is exhausted to waste through conduit 7, and then the particles are released from trap $M_2$ and flowed in a suitable fluid through conduits 7 and 8 to counter S.

It will be understood that Figure 2 is a simplified picture only. Further details of the use of magnetic particles in continuous flow assays are given in our U.S. patent no. 4141687 to which reference should be made for further details.

In order that the invention may be more fully understood, the following Example is given by way of illustration only.

- 11 -

EXAMPLE

Manual assay of thyroxine

200 µl of a serum sample containing, as analyte, thyroxine was mixed with 200 µl of a suspension of magnetically attractable particles in a clear polystyrene test-tube. The suspension of particles contained 2 mg of particles, of average size 5 microns and specific gravity closely similar to that of the liquid in the mixture. The particles comprised cellulose (with iron oxide therein), the cellulose having been treated with cyanogen bromide and then bound to anti-thyroxine antibody as receptor.

Merthiolate (2.4 mg) was included in the mixture to displace thyroxine from its serum binding proteins. Methocel (1.5 mg) was included to retard sedimentation of the suspended particles so that continuous mixing was not necessary. Triton detergent was included in all buffers to prevent or reduce nonspecific binding and also to enhance the fluorescence of the labelled thyroxine.

The mixture was incubated at room temperature for 30 minutes with a vortex mix after 15 minutes.

After the addition of 1ml of bicarbonate buffer (pH 8.6, containing 0.1% Triton), the solid phase was sedimented using a permanent magnet and the supernatant liquid was aspirated to waste. Fluorescein-labelled thyroxine (400 nanograms in 200 µl) was added to the solid phase in the test-tube and mixed. Methocel (2 mg) was again included to maintain the suspension. The mixture was incubated at room temperature for 30 minutes with a vortex mix after 15 minutes.

Bicarbonate buffer (1 ml) was added, and the particles were then sedimented using a magnet. The fluorescence of the clear supernatant liquid ("free fraction" of the labelled thyroxine) was then measured by placing the test-tube in a suitably modified sample compartment of a fluorimeter. The sedimented solid phase was clear of the exciting light beam and did not interfere in the end point measurement.

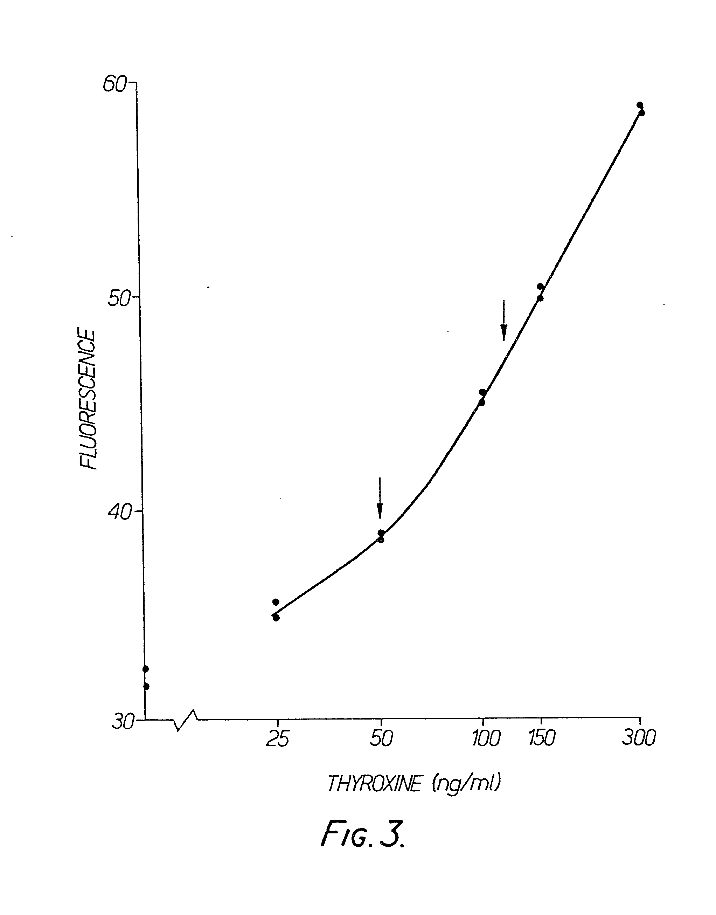

The assay was repeated several times using different known concentrations of thyroxine, and a standard curve constructed (Figure 3) showing the relation between thyroxine concentration and fluorescence. On Figures 3, the two arrows represent the usual limits of normal thyroxine levels.

As stated previously, the method of the invention can be carried out on a manual discrete basis, or in an automated continuous flow manner. For the purposes of manual assays, the reagent means for carrying out the method can be provided in the form of a kit. Thus, the invention includes a kit for carrying out the method, the kit comprising

    a)    a quantity of magnetically attractable particles comprising a receptor which is reactive with the analyte,

    b)    labelled analyte; and

    c)    analyte in the form of, or to be made into, solutions of known concentration to serve as standards.

Any, or all three of a), b) and c) can be in lyophilised form. The kit may also include a solution of, or lyophilised, analyte in control serum.

CLAIMS:

1.      A method of quantitatively assaying a liquid
for an analyte therein which comprises the steps of (a)
mixing a sample of the liquid with finely divided magnetically
attractable particles comprising a reagent, and then (b)
separating the particles from the mixture,
Characterised in that:

i)      the said reagent is a receptor which selectively
        binds with the analyte in the mixture, the amount
        of receptor used in step (a) being in excess of that
        required to bind with the analyte in the mixture;

ii)     the particles separated in step (b) have analyte
        bound thereto;  and

iii)    the method includes the further step
        (c)  assaying the receptor excess or the amount
             of analyte bound to the particles and therefrom
             determining the amount of analyte in the
             liquid sample.

2.      A method according to claim 1, characterised in
that step (c) comprises forming a suspension of the separated particles from step (b) in a liquid comprising a
labelled substance which will bind with the excess receptor
or with the analyte, but not with both, and then measuring
the amount of label bound to the particles or remaining
unbound in the liquid.

3.      A method according to claim 2, characterised in
that after addition of the labelled substance, the particles
are removed from at least a portion of the suspension, and
the amount of labelled substance remaining in the liquid
in said portion is determined.

4.          A method according to claim 3, which is effected on
a discrete basis, <u>characterised in that</u> in step (c) the suspension of particles is formed in a vessel and the particles
are magnetically held localised in a region of the vessel
to provide said portion free from particles in the vessel.

5.          A method according to claim 4 or 5, <u>characterised</u>
<u>in that</u> in step a) the sample and the particles are mixed
in a vessel;  in step b) the particles are held within the
vessel by magnetic means whilst the liquid is aspirated
or decanted from the vessel;  and the suspension of step c)
is formed in the same said vessel.

6.          A method according to claim 2,3,4 or 5 <u>characterised</u>
<u>in that</u> the labelled substance is coloured or fluorescent
and the amount of said substance remaining unbound in the
liquid is measured by spectrophotometry or by fluorimetry,
respectively.

7.          A method according to claim 1 to 2, <u>characterised</u>
<u>in that</u> it is effected on a continuous automated basis by
continuous flow techniques.

8.          A method according to claim 3, <u>characterised in</u>
<u>that</u> it is effected on a continuous automated basis by
continuous flow techniques, wherein the mixture formed
in step a) is flowed along a conduit;  the particles are
separated from the mixture in step b) by magnetically traping
them to hold them against flow of the liquid, thereby to
separate them from the liquid;  the suspension of step c)
is formed within said conduit and flowed therealong, and
wherein said portion of liquid free from particles is formed
from said suspension by holding said particles in a magnetic
trap, the said portion of liquid being assayed for the
labelled substance.

9.      A method according to claim 8, <u>characterised</u> <u>in</u> <u>that</u> the labelled substance is coloured or fluorescent.

10.      A method according to any preceding claim, <u>characterised</u> <u>in</u> <u>that</u> the analyte is an antigen or hapten, and the said receptor is an antibody or other binding protein.

11.      A method according to claims 3 and 10, <u>characterised</u> <u>in</u> <u>that</u> the labelled substance is labelled antigen or hapten under assay, or a closely similar labelled substance.

12.      A method according to any preceding claim, <u>characterised</u> <u>in</u> <u>that</u> the analyte is an antibody or other binding protein and the receptor is an antigen or hapten.

13.      A method according to claims 3 and 12, <u>characterised</u> <u>in</u> <u>that</u> the labelled substance is labelled antibody or other binding protein, or a closely similar labelled substance.

14.      A method according to claim 10 or 11, wherein the analyte is a thyroid hormone or human placental lactogen.

15.      Apparatus for carrying out the method of claim 8, which comprises means for flowing a plurality of reaction mixtures in turn along a conduit on a discrete basis; first trap means (M1) for magnetically trapping the particles in the conduit in each reaction mixture to hold them against the flow and so separate them from their respective mixture; second magnetic trap means (M2) downstream of the said first trap means;  and assay means (S) downstream of the second trap means;  <u>characterised</u> <u>in</u> <u>that</u>  means are provided for mixing the particles separated in the first trap means with a labelled substance, and wherein the said assay means is arranged to assay the label.

16.      A kit for carrying out manually the method of claim 3, which is <u>characterised in that</u> it comprises:

i)      a quantity of magnetically attractable particles comprising a receptor which is reactive with the analyte to be assayed to selectively bind thereto;

ii)     a quantity of labelled analyte to form the liquid comprising a labelled substance in step (c) of the said method;

iii)    analyte in the form of, or to be made into, solutions of known concentration to serve as standards;  and

iv)     instructions as to the performance of the assay; the kit being packaged in unitary form.

17.      A kit according to claim 16, <u>characterised in that</u> one or more of components (i) to (iii) are in lyophilised form.

*Fig. 1.*

*Fig. 2.*

Fig. 3.

European Patent Office

**EUROPEAN SEARCH REPORT**

Application number

EP 80 30 3993.2

| | DOCUMENTS CONSIDERED TO·BE RELEVANT | | CLASSIFICATION OF THE APPLICATION (Int. Cl.³) |
|---|---|---|---|
| Category | Citation of document with indication, where appropriate, of relevant passages | Relevant to claim | |
| | DE - A1 - 2 710 438 (TECHNICON INSTRU-MENTS CORP.)<br>* claims 1 to 5 and 24 to 43 *<br>--<br>US - A - 4 115 534 (D.S. ITHAKISSIOS)<br>* example 1 *<br>-- | 1-3,<br>7-13,15<br><br><br>1,2 | G 01 N 33/54 |
| A | DE - A1 - 2 641 713 (BIO-RAD LABORATORIES INC.)<br>* pages 13 to 15 *<br>-- | 1 | |
| A | GB - A - 2 005 019 (TECHNICON INSTRU-MENTS CORP.)<br>-- | | TECHNICAL FIELDS SEARCHED (Int. Cl.³)<br><br>G 01 N 33/54 |
| A | CLINICAL CHEMISTRY, Vol. 23, No. 11, 1977, Washington<br>D.S. ITHAKISSIOS et al. "Use of Protein Containing Magnetic Microparticles in Radioassays"<br>pages 2072 to 2079<br>---- | | |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family corresponding document

| X | The present search report has been drawn up for all claims | | |
|---|---|---|---|
| Place of search<br>Berlin | Date of completion of the search<br>18-02-1981 | Examiner<br>SCHWARTZ | |

EPO Form 1503.1 06.78